Europäisches Patentamt

**European Patent Office**

**Office européen des brevets**

(19)

(11) Publication number: **0 094 123**
A2

## (12) EUROPEAN PATENT APPLICATION

(21) Application number: **83200627.4**

(51) Int. Cl.³: **A 61 K 9/54,** A 61 K 9/16

(22) Date of filing: **03.05.83**

(30) Priority: **06.05.82 US 375426**
**18.04.83 US 485993**

(43) Date of publication of application: **16.11.83**
**Bulletin 83/46**

(84) Designated Contracting States: **AT BE CH DE FR GB IT LI NL SE**

(71) Applicant: **THE PROCTER & GAMBLE COMPANY, 301 East Sixth Street, Cincinnati Ohio 45202 (US)**

(72) Inventor: **Wahl, Errol Hoffman, 8021 Deershadow Lane, Montgomery, OH 45242 (US)**

(74) Representative: **Suslic, Lydia et al, Procter & Gamble European Technical Center Temselaan 100, B-1820 Strombeek-Bever (BE)**

(54) **Therapeutic granules.**

(57) Enteric coated therapeutic granules are disclosed which have a first coating of a therapeutic agent over the therapeutic core and an enteric coating adhered to the therapeutic coating.

EP 0 094 123 A2

## THERAPEUTIC GRANULES

Errol H. Wahl

### TECHNICAL FIELD

The present invention is related to therapeutic granules which have an enteric coating.

The use of therapeutic agents such as aspirin is widespread as a means for alleviating pain of various types. One problem associated with aspirin for many people is irritation of the stomach. As a result prior art developments have provided enteric coatings which allow the aspirin particles to pass through the stomach and into the intestines. The more alkaline environment of the intestines dissolves/disrupts the enteric coating allowing the aspirin to be released. Insuring satisfactory coatings has been a problem continually faced by workers in the field.

### BACKGROUND OF THE INVENTION

As noted above enteric coatings for analgesics have been disclosed in the prior art.

Specific disclosures concerning enteric coatings are contained in many different patents. U.S. Patent 3,524,910, August 18, 1970 to Holliday et al discloses analgesic compositions wherein the analgesic (e.g. aspirin) is coated with ethyl cellulose in a weight amount relative to the amount of analgesic of from 1 – 22 to 1 – 50. U.S. Patent 3,656,997, April 18, 1972 to Cordes discloses analgesic containing gelatin capsules having a first coating of an enteric material. U.S. Patent 3,691,090, September 12, 1972 to Kitajima et al discloses a method of encapsulating aspirin cores with an enteric polymer. U.S. Patent 3,906,086, September 16, 1975 to Guy et al discloses particles having an aspirin core and an enteric phthalate coating. U.S. Patent 4,308,251, December 29, 1981, to Dunn et al discloses aspirin tablets containing aspirin, an enteric material and an erosion promoting agent such as corn starch.

Additional references disclosing coatings on analgesics include U.S. Patent 2,953,497, September 20, 1960 to Press which discloses analgesic granules having a cellulose acetate phthalate coating; U.S. Patent 3,166,476, January 19, 1965 to Lowey which discloses aspirin particles which have a first coating of a material such as gelatin and a second enteric coating; British Patent 1,129,811, October 9, 1968 to Aspro-Nicholas Ltd. which discloses aspirin particles which have a coating of a polysalicylide and possibly a second, unspecified, coating.

While the prior art discloses a variety of enteric coated therapeutic approaches, areas for improvement still exist.

It has now been discovered that providing a first coating of a therapeutic over the therapeutic core provides for more efficient application of the enteric coating.

It is therefore an object of the present invention to provide therapeutic granules which have a more efficient enteric coating.

It is a further object of the present invention to provide therapeutic granules which have a first therapeutic coating and a second enteric coating.

It is still a further object of the present invention to provide a method for treating pain, fever, and inflammation.

These and other objects will become readily apparent from the detailed description which follows. All percentages and ratios herein are by weight unless otherwise specified.

0094123

- 3 -

## DESCRIPTION OF THE INVENTION

The present invention pertains to enteric coated therapeutic granules comprising:

(a)  a nonsteroidal, therapeutic active core;

(b)  a first coating adhered to said core of a nonsteroidal, therapeutic active; and

(c)  a second coating adhered to said first coating of an enteric material;

wherein said enteric coated granules are from about 0.5mm to about 1.5mm, preferably from about 0.5 mm to about 1.2 mm, in diameter, the weight ratio of said core to said first coating is from about 50:1 to about 1:1 and said enteric coating is from about 1% to 20% by weight of the entire enteric coated granules.

The present invention also encompasses a method for alleviating pain, fever, and inflammation. The essential as well as optional components of the invention claimed herein are set forth in the paragraphs which follow. In the present application the following terms have the meanings given.

"Pharmaceutically-acceptable" or "pharmacologically-acceptable", as used herein, means that the ingredients used in the compositions are suitable for use in contact with the tissue of humans, without undue toxicity, irritation, allergic response, and the like, commensurate with a reasonable benefit/risk ratio.

The term "comprising", as used herein, means that various other compatible components, including both active and inert ingredients, can be conjointly employed in the compositions of this invention. The term "comprising" thus encompasses and includes the more restrictive terms "consisting of" and "consisting essentially of".

By "compatible" herein is meant that the components of the present invention are capable of being commingled without interacting in a manner which would substantially decrease the efficacy of the therapeutic under ordinary use conditions.

All percentages and ratios for the components are set forth hereinbelow.

## NONSTEROIDAL THERAPEUTIC ACTIVE CORE

The core of the granules described and claimed herein is a nonsteroidal, therapeutic active.

The therapeutic actives used in the present invention preferably have analgesic, antipyretic and anti-inflammatory properties. Aspirin is an example of such a material and is the preferred material. However, there are numerous and other materials which can be used. These include calcium carbaspirin, chloline salicylate, salicylic acid, naproxen, ibuprofen, fenoprofen, zomepirac and mixtures thereof.

The core of the present granules generally contains about 50% to about 100%, preferably from about 85% to about 95% of the therapeutic active.

Optional components which may be used in the cores include diluents, binders, disintegrents, lubricants, glidents, buffering adjuvants and direct acting adjuvants. Numerous pharmaceutically acceptable and compatible materials can be found in various pharmacological references. Two such references are Pharmaceutical Dosage Forms: Tablets, Vol. 1, Ed. H. A. Lieberman and L. Lachman, Mercel Dekker, Inc., 1980 and "Proposed Monograph for OTC Internal Analgesic, Antipyretic and Antirheumatic Drugs", Federal Register, July 8, 1977. Both are incorporated herein by reference.

Exemplary materials of the above types include dicalcium phosphate and lactose as diluents; gelatin and polyvinylpyrrolidone as binders; starch, sodium starch glycolate and cellulosics as disintegrents; stearates as lubricants; fumed silica as glidents; glycine and other amino acids, bicarbonate salts, carbonate salts, magnesium and aluminum hydroxides, aluminum glycinate, citrate salts and borate salts as buffering adjuvents;

and caffeine, ascorbic acid and other vitamins, antihistamines and other cough, cold, allergy bronchodilator and antiasthamatic drugs as direct acting adjuvents.

These optional components individually may be present at a level of from about 0% to about 50%, preferably from about 5% to about 15% of the core.

Active Coating

The active coat which is adhered to the core of the granules can be any of the actives mentioned previously as suitable for core use. Additionally, other materials such as acetaminophen can be used. It should also be recognized that the active coat may be a different drug than the core but is preferably the same. Aspirin is the preferred material.

The weight ratio of the core to the first (active) coat is from about 50:1 to about 1:1, preferably from about 2:1 to about 1:1. Amounts of coating within these ranges allow for adequate covering of the rough core surface while not making the total granule excessively large.

Included among optional components in the first coat, as set forth in the discussion of the core, are binders, disintegrents, buffers and direct acting adjuvants. An amount of said optional components, individually, found useful is from about 4% to about 80%, preferably from about 6% to about 20% by weight of the active coating.

Enteric Coating

The materials useful as the enteric coating for the present granules include any pharmaceutically acceptable material insoluble at pH's of from about 1 to about 3 while being soluble at pH's in the range of from about 4.5 to about 8.0. Suitable materials include hydroxypropyl methylcellulose phthalate, cellulose acetate phthalate, acrylic anionic polymers and polyvinyl acetyl phthalate. Various fats and oils are also satisfactory as are mixtures of the foregoing. The enteric coating is present at a level of 1% to about 20%, preferably from about 4% to about 12%, most preferably from about 8% to about 10% of the entire enteric coated granule. The preferred coatings are hydroxypropyl methylcellulose phthalate and polyvinyl acetylphthalate.

Optional components suitable for combining with the enteric material include surface active agents and plasticizers such as triethyl citrate, triacetin, triglycerides and acetylated monoglyceride.

## METHOD OF MANUFACTURE

The following procedure can be used to make granules of the type disclosed and claimed herein.

Commercially available (Dow Chemical Co.) aspirin starch granules (90% aspirin/10% starch) are screened to provide granules with a diameter range of 0.5 to 1.0mm. Said granules are then coated with the therapeutic coating using a Wurster fluidized bed coater (Glatt Air Techniques, Inc., Model WSG5) with a seven inch column diameter.

Two thousand grams of sieved granules are placed in the Wurster coater. The therapeutic coat components (Example 1) are slurried with a 1:1.5 weight:volume ratio of isopropyl alcohol and maintained in a suspended state during the coating application via agitation. The coating slurry is applied at a rate of 140 ml/min. through a 1.0 mm nozzle and atomized with 60 1/min. air at 90 psig. The bed is fluidized with 2180 1/min. air at 52°C. inlet temperature. The exit air temperature under these conditions is about 30°C. The coating operation is continued until 4000 ml (1500g solids) of therapeutic coating are applied.

The therapeutically coated granules are then sieved to a particle size range of 0.5 to 1.2 mm and 1900g are placed in the Wurster coater. An 8.0% wt./vol. solution of the enteric coat components (Example 1) in an azeotrope mixture of ethyl acetate and isopropyl alcohol (75:25 wt.:wt.) is then prepared. The coating solution is applied at a rate of 40 ml/min. through a 0.8 mm nozzle and atomized with 70 1/min. air at 90 psig. The bed is fluidized with 2180 1/min. air at 63°C. inlet temperature. The exit air temperature is about 40°C. under these conditions. The coating operation is continued until 1250 ml of solution (100g solids) are applied.

The enteric coated particles are then sieved to a size range of 0.5 to 1.2 mm and formulated into the final dosage form.

## FIELD OF USE

The granules of the present invention can be used in tablets, capsules or in any other convenient form. The therapeutic agents are well recognized for treating a wide variety of human ailments.

The following non-limiting examples illustrate the compositions of the present invention.

| Core | EXAMPLES I - IV | | | | % of total granule |
|---|---|---|---|---|---|
| | I (54.3%) | II (50.5%) | III (52.2%) | IV (54.4%) | |
| Aspirin | 90.0 | 94.0 | 90.0 | 90.0 | |
| Starch | 10.0 | | | | |
| Crosslinked carboxy-methyl cellulose | | 3.0 | | | |
| Sodium starch glycolate | | | 5.0 | 5.0 | |
| NaHCO$_3$ | | | 5.0 | | |
| Ascorbic Acid | | 3.0 | | 3.0 | |
| Polyvinyl pyrrollidone | | | | 2.0 | |
| | 100.00 | 100.00 | 100.00 | 100.00 | |

| Active Coat | (40.7%) | (45.5%) | (41.8%) | (38.1%) | % of total granule |
|---|---|---|---|---|---|
| Aspirin | 94.0 | 92.0 | 95.0 | 85.0 | |
| Polyvinyl pyrrollidone | 4.0 | 3.0 | 3.0 | 5.0 | |
| Sodium starch glycolate | 2.0 | | 2.0 | | |
| Starch | | 5.0 | | 10.0 | |
| | 100.00 | 100.00 | 100.00 | 100.00 | |

EXAMPLES I - IV (continued)

| Enteric Coat | I (5.0%) | II (4.0%) | III (6.0%) | IV (7.5%) | % of total granule |
|---|---|---|---|---|---|
| Hydroxypropyl methylcellu- lose phthalate | 90.0 | 75.0 | | 90.0 | |
| Cellulose acetate phthalate | | | 90.0 | . | |
| Acetylated monoglycerides | 10.0 | | 10.0 | 10.0 | |
| Triethyl citrate | | 25.0 | | | |
| | 100.00 | 100.00 | 100.00 | 100.00 | |

- 10 -

The above compositions are capable of reducing aspirin contact with the acidic environment of the stomach while still providing satisfactory release of the aspirin in the intestines.

CLAIMS

1. Therapeutic enteric coated granules characterized in that they comprise:

   (a) a nonsteroidal, therapeutic active core;

   (b) a first coating adhered to said core of a nonsteroidal, therapeutic active; and

   (c) a second coating adhered to said first coating of an enteric material;

   wherein said enteric coated granules are from 0.5mm to 1.5mm in diameter the weight ratio of said core to said first coating is from 50:1 to 1:1 and said enteric coating is from 1% to 20% by weight of the entire coated granule.

2. Enteric coated granules according to Claim 1 characterized in that the therapeutic active core is selected from the group consisting of aspirin, calcium carbaspirin, chloline salicylate, magnesium salicylate, sodium salicylate, salicylic acid, naproxen, ibuprofen, fenoprofen, zomepirac and mixtures thereof.

3. Enteric coated granules according to Claim 2 characterized in that the first coating is selected from the group consisting of aspirin, calcium carbaspirin, chloline salicylate, magnesium salicylate, sodium salicylate, salicylic acid, naproxen, ibuprofen, fenoprofen, zomepirac, acetaminophen, and mixtures thereof.

4. Enteric coated granules according to Claim 3 characterized in that the enteric coating is selected from the group consisting of hydroxypropyl methylcellulose phthalate, cellulose acetate phthalate, acrylic anionic polymers, polyvinyl acetylphthalate, fats, oils and mixtures thereof.

5. Enteric coated granules according to Claim 4 characterized in that the size of the granules is from 0.5 mm to 1.2 mm.

6. Enteric coated granules according to Claim 5 characterized in that the first coating is selected from the group consisting of aspirin, acetaminophen and mixtures thereof.

7. Enteric coated granules according to Claim 6 characterized in that the enteric coating is selected from the group consisting of hydroxypropyl methyl cellulose phthalate, polyvinyl acetylphthalate and mixtures thereof.

8. Enteric coated granules according to Claim 7 characterized in that the therapeutic active core is aspirin, the first coating is aspirin and the enteric coating is hydroxypropyl methylcellulose phthalate.

9. Enteric coated granules according to Claim 8 characterized in that the weight ratio of said core to said first coating is from 2:1 to 1:1.

10. Enteric coated granules according to Claim 9 characterized in that the amount of the enteric coating is from 4% to 12% of the entire coated granule.